# FASCICULE DE BREVET EUROPEEN

(11) **EP 2 150 222 B1**
(45) Date de publication et mention de la délivrance du brevet: **17.10.2012**
(21) Numéro de dépôt: 08805593.4
(22) Date de dépôt: 21.05.2008
(51) Int. Cl.: A61F 9/007

(54) **DISPOSITIF POUR LE TRAITEMENT DU LARMOIEMENT DE L'OEIL**
VORRICHTUNG ZUR BEHANDLUNG VON TRÄNENDEM AUGE
DEVICE FOR TREATMENT OF WATERING OF THE EYE

(30) Priorité: 22.05.2007 FR 0703626
(43) Date de publication de la demande: 10.02.2010
(73) Titulaire: Optisoins SA, 1950 Sion (CH); Sarfati, Eric, 83200 Toulon (FR)
(72) Inventeur: SARFATI, Eric, F-83200 Toulon (FR)
(74) Mandataire: Maureau, Philippe
(86) Numéro de dépôt international: PCT/FR2008/000702
(87) Numéro de publication internationale: WO 2009/004140

(56) Documents cités:
- WO-A-2006/039096
- DE-A1- 19 823 755
- GB-A- 484 499
- US-A- 6 083 188

## Description

La présente invention porte sur une sonde, monocanaliculaire ou bicanaliculaire, utilisable dans le traitement du larmoiement de l'oeil.

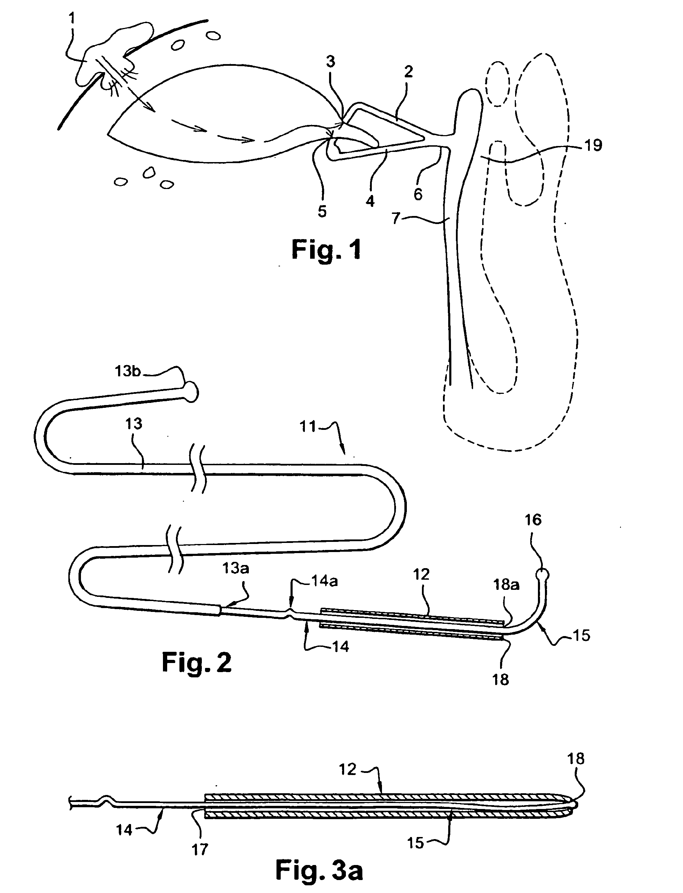

Le larmoiement de l'oeil peut être dû à une trop grande production de larmes par la glande lacrymale. Dans ce cas, le traitement agit sur la glande pour réduire sa sécrétion. Une autre cause du larmoiement de l'oeil est l'obstruction, ou sténose, des voies lacrymales excrétrices, à savoir le canalicule supérieur, le canalicule inférieur, le canalicule d'union et le canal lacrymo-nasal. Les canalicules correspondent avec l'oeil par des méats disposés au voisinage du nez dans les paupières supérieures et inférieures et ils ont pour fonction d'évacuer le liquide lacrymal par le nez. La figure 1 illustre les voies lacrymales de l'oeil droit d'un patient. Sur cette figure sont représentés la glande lacrymale 1, le canalicule supérieur 2 et le méat supérieur 3, le canalicule inférieur 4 et le méat inférieur 5, le canalicule d'union 6 et le canal lacrymo-nasal 7. Lorsque l'obstacle obstruant un de ces canaux est franchissable par une sonde, on cathétérise ces voies et on introduit une sonde, généralement formée d'un élément filiforme, par exemple un tube de silicone, le long duquel les larmes pourront être évacuées vers le nez, rétablissant ainsi la circulation lacrymale.

Une technique connue d'intubation utilise une sonde comprenant un élément filiforme dont chaque extrémité est munie d'un mandrin métallique rectiligne qui sert de guide à l'élément filiforme. Dans cette méthode, le mandrin métallique de la première extrémité est par exemple introduit dans le canalicule inférieur par le méat inférieur puis il bifurque dans le canal lacrymo-nasal puis est récupéré dans la fosse nasale. Dans un deuxième temps, le mandrin métallique de la deuxième extrémité est introduit dans le canalicule supérieur par le méat supérieur puis il bifurque dans le canal lacrymo-nasal et est récupéré dans la fosse nasale. L'inconvénient de cette méthode est qu'il est particulièrement difficile de récupérer chacun des mandrins dans les fosses nasales. Cette opération, répétée pour chaque mandrin, se fait souvent à l'aveugle à l'aide d'une pince et elle est particulièrement traumatisante pour les chairs qui sont abîmées et qui saignent.

Une autre méthode connue d'intubation utilise une canule creuse dans laquelle on fait glisser l'élément filiforme. Ainsi, dans un premier temps, la canule est introduite dans le canalicule inférieur par le méat inférieur puis elle bifurque dans le canal lacrymo-nasal. La première extrémité de l'élément filiforme est alors glissée à l'intérieur de la canule puis est récupérée dans la fosse nasale. On procède de la même façon avec la deuxième extrémité et le canalicule supérieur. Toutefois, cette méthode présente également l'inconvénient d'avoir à récupérer les extrémités de l'élément à l'aveugle à l'aide d'une pince ou d'un crochet dans la fosse nasale. Cette opération est particulièrement sanglante et traumatisante.

GB 484,499 décrit un dispositif selon le préamble de la revendication 1.

La présente invention vise à remédier à ces inconvénients.

Un objectif de la présente invention est de fournir un dispositif, tel qu'une sonde, monocanaliculaire ou bicanaliculaire, permettant la récupération atraumatique de la ou des extrémités dudit dispositif au niveau de la ou des fosse(s) nasale(s).

La présente invention porte sur un dispositif pour le traitement du larmoiement de l'oeil, comprenant un élément filiforme et une canule d'intubation, caractérisé en ce qu'au moins une extrémité de l'élément filiforme est munie d'une tige en métal élastique comprenant une extrémité distale courbée, ladite tige en métal élastique étant agencée pour coulisser au sein de ladite canule d'intubation.

Grâce à la nature élastique de la tige en métal du dispositif selon l'invention et à sa forme courbée, l'extrémité de la tige en métal sort automatiquement de la canule dans la fosse nasale : pour cela, il suffit que le chirurgien pousse simplement sur l'élément filiforme ou sur la partie proximale de la tige au niveau de l'extrémité proximale de la canule. L'extrémité distale courbée de la tige en métal du dispositif selon l'invention est visible tout de suite par le chirurgien qui n'a ainsi pas besoin d'aller la chercher de façon hasardeuse avec une pince ou un crochet. Les chairs, notamment les tissus nasaux, ne sont pas abîmés et l'opération est beaucoup moins traumatisante pour le patient. Une telle opération peut ainsi s'effectuer en cabinet.

Dans la présente demande, on entend par extrémité distale d'une pièce l'extrémité la plus éloignée de l'utilisateur du dispositif et par extrémité proximale, l'extrémité la plus proche de l'utilisateur du dispositif.

De préférence, l'élément filiforme est un tube de silicone. De façon non limitative, tout matériau souple susceptible de drainer les larmes pourrait avantageusement remplacer le silicone pour constituer l'élément filiforme.

Dans une forme de réalisation de l'invention, la canule d'intubation peut être courbée.

Dans une forme préférée de réalisation de l'invention, la tige en métal élastique présente une portion rectiligne et une portion courbée, ladite portion courbée comprenant l'extrémité distale courbée de la tige.

Dans une forme préférée de réalisation de l'invention, l'extrémité distale de la tige en métal élastique est courbée en arc de cercle.

Dans un mode particulier de réalisation de l'invention, ladite extrémité distale courbée de la tige présente un rayon de courbure de 1 cm. De façon plus particulière, ladite extrémité distale courbée de la tige forme un arc de cercle allant de un quart à trois quarts de cercle.

Dans une forme préférée de réalisation de l'invention, la tige en métal élastique est constituée d'un acier inoxydable, par exemple d'un acier inoxydable présentant des propriétés super élastiques comme l'acier inoxydable vendu sous la dénomination commerciale « 304 » par la société UGINE. Par métal ou alliage présentant des propriétés super élastiques, on entend au sens de la présente demande tout métal ou alliage présentant une possibilité de déformation purement élastique de 5 à 10 fois supérieure, voire 20 fois supérieure, à celle de l'acier.

Dans une autre forme de réalisation de l'invention, la tige en métal élastique est constituée d'un alliage à base de cobalt, de chrome, de nickel et de molybdène.

Grâce à l'élasticité du métal ou de l'alliage formant la tige en métal du dispositif selon l'invention, cette tige peut d'une part se déformer sous la contrainte exercée sur elle par les parois de la canule tendant à aligner son extrémité distale courbée selon l'axe longitudinal de la canule, lorsqu'elle est au sein de ladite canule, et d'autre part reprendre sa forme initiale lorsque cette contrainte a cessé, c'est-à-dire à la sortie de la canule.

De préférence l'élément filiforme, de préférence un tube en silicone, a une longueur allant de 20 à 50 cm, de préférence allant de 20 à 30 cm.

Avantageusement, la tige en métal élastique a une longueur totale d'environ 18 cm. De façon alternative, la tige a une longueur totale d'environ 15 cm.

Dans une forme de réalisation du dispositif selon l'invention, ladite canule d'intubation est solidaire de ladite tige en métal élastique. Ainsi, de préférence, l'extrémité distale courbée de la tige en métal présente à son extrémité distale une excroissance de diamètre supérieur au diamètre interne de l'orifice distal de la canule d'intubation.

Dans une forme de réalisation du dispositif selon l'invention, ladite tige en métal élastique comprend dans sa partie proximale un décrochement orienté dans la même direction que la courbure de l'extrémité distale courbée de ladite tige. Ce décrochement constitue un repère pour le chirurgien, lui indiquant dans quelle direction l'extrémité distale courbée de la tige en métal va sortir de la fosse nasale.

Dans une telle forme de réalisation du dispositif selon l'invention, le coulissement de ladite tige au sein de la canule d'intubation est limité par ladite excroissance de l'extrémité distale courbée et par ledit décrochement. La tige en métal élastique est agencée pour coulisser au sein de ladite canule entre ladite excroissance et ledit décrochement seulement. La tige est ainsi solidaire de la canule d'intubation.

Dans une forme de réalisation de l'invention, chacune des deux extrémités de l'élément filiforme, de préférence un tube en silicone, est munie d'une tige en métal élastique. Le dispositif selon l'invention est alors une sonde bicanaliculaire.

Dans le cas où seule une extrémité de l'élément filiforme est munie de ladite tige en métal élastique, le dispositif selon l'invention est une sonde monocanaliculaire.

La pose d'un dispositif selon l'invention comprend les étapes suivantes :
- a°) on introduit la canule d'intubation, dans laquelle est glissée la tige en métal élastique d'une extrémité de l'élément filiforme du dispositif selon l'invention, par un méat dans un canalicule puis dans le canal lacrymo-nasal. Dans le cas où l'on utilise une tige en métal élastique munie d'un décrochement, il est préférable de bien positionner la canule d'intubation en contrôlant que le décrochement se situe vers le haut ;
- b°) on pousse sur l'élément filiforme ou sur la partie proximale de la tige en métal dans le sens distal, au niveau de extrémité proximale de la canule, jusqu'à ce que l'extrémité distale courbée de la tige en métal élastique sorte de la canule automatiquement au niveau de la fosse nasale. La courbure de la tige en métal élastique et le contact avec la paroi inférieure du nez font sortir la tige en métal élastique au niveau de la partie antérieure nasale. Il est préférable, après la mise en oeuvre de l'étape a°) et avant la mise en oeuvre de l'étape b°), de retirer la canule de 1 cm environ.- c°) on saisit, par exemple avec le doigt, l'extrémité distale courbée de la tige en métal élastique au niveau de la fosse nasale et on tire dessus pour engager parfaitement l'élément filiforme dans le trajet lacrymal. Lors de la mise en oeuvre de cette étape, on peut également saisir la canule avec, par exemple, une pince ou un crochet.

Dans le cas où le dispositif selon l'invention est une sonde bicanaliculaire, les deux extrémités de l'élément filiforme sont munies d'une tige en métal élastique. Les deux extrémités de l'élément filiforme d'une sonde bicanaliculaire sont préférentiellement munies d'une canule d'intubation. Dans un tel cas, la pose du dispositif comprend la répétition des étapes a°) à c°) ci-dessus à partir du deuxième méat dans le deuxième canalicule avec la deuxième tige en métal élastique située à la deuxième extrémité de l'élément filiforme du dispositif.

Ainsi, grâce à l'invention, il suffit de pousser simplement sur l'élément filiforme ou sur la partie proximale de la tige en métal lors de l'intubation pour que l'extrémité distale courbée de la tige en métal, grâce à son élasticité et sous les contraintes des parois osseuses nasales, sorte automatiquement dans la bonne direction dans la fosse nasale puis hors de cette fosse. Préférentiellement, il suffit de pousser sur la partie proximale de la tige en métal élastique, par exemple à distance du décrochement le cas échéant, et à proximité de la partie proximale de la canule.

D'autres caractéristiques et avantages de l'invention apparaîtront au cours de la description qui va suivre d'un mode particulier de réalisation, donné uniquement à titre d'exemple non limitatif en regard des dessins qui représentent :
- la figure 1 est une vue en coupe des voies lacrymales de l'oeil droit d'un patient ;
- la figure 2 est une vue en coupe d'une sonde monocanaliculaire selon l'invention ;
- la figure 3a est une vue en coupe partielle du dispositif selon l'invention dans laquelle l'extrémité distale courbée de la tige en métal est sous contrainte au sein de la canule d'intubation ;
- la figure 3b est une vue de côté d'un mode de réalisation particulier de la tige en métal élastique du dispositif selon l'invention ;
- la figure 3c est une vue de détail de la figure 3b ;
- la figure 3d est une vue en coupe partielle d'un mode de réalisation d'un dispositif selon l'invention dans lequel l'extrémité proximale de la tige en métal élastique est sertie dans l'élément filiforme au moyen d'un système de sertissage ;
- la figure 3e est une vue en coupe partielle d'un mode de réalisation de l'invention dans lequel la canule d'intubation est courbée ;
- la figure 4 illustre le trajet de la canule d'intubation depuis son introduction dans un méat jusqu'à sa sortie dans la fosse nasale ;
- la figure 5 représente la canule d'intubation en place avec l'extrémité distale courbée de la tige en métal est sous contrainte au sein de la canule d'intubation ;
- les figures 6 à 8 montrent la mise en place du dispositif selon l'invention.

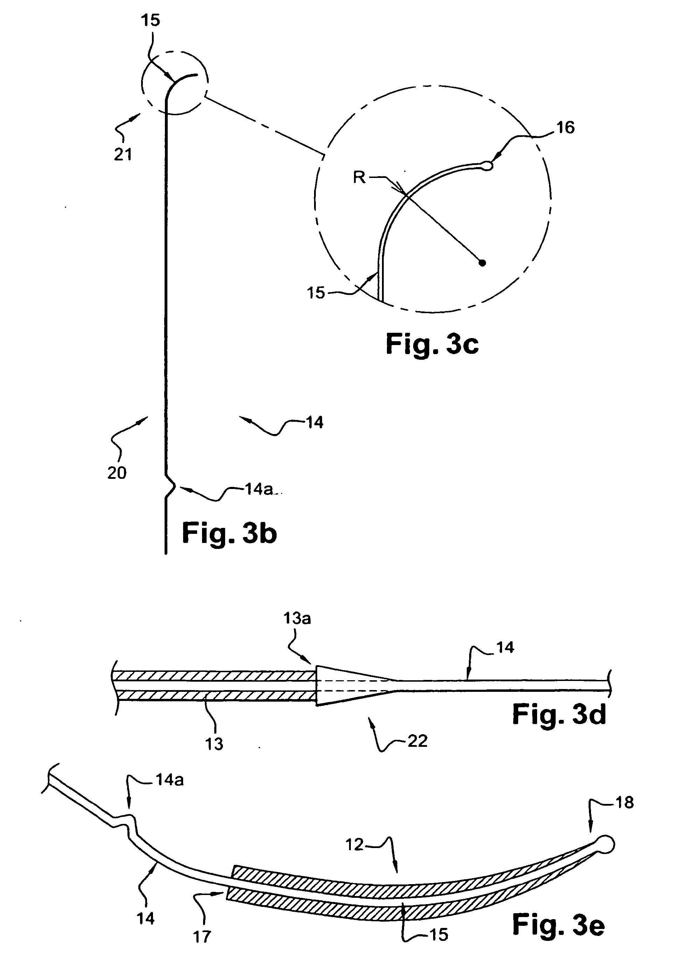

Sur les figures 2 et 3a sont représentés un dispositif 11 pour le traitement du larmoiement de l'oeil, sous la forme d'une sonde monocanaliculaire. Le dispositif 11 représenté sur ces figures comprend un élément filiforme 13, de préférence un tube en silicone, et une canule d'intubation 12.

Une extrémité 13a de l'élément filiforme 13 est munie d'une tige 14 en métal élastique comprenant une extrémité distale courbée 15. L'autre extrémité 13b de l'élément filiforme est munie d'un bouchon méatique 13c, par exemple en silicone ou en acrylique.

Par exemple, la tige 14 en métal élastique est reliée à l'élément filiforme 13, en particulier au tube de silicone, par collage ou sertissage.

La figure 3d décrit un système de sertissage 22, à l'extrémité proximale de la tige 14 en métal élastique, dont le diamètre varie régulièrement et progressivement, de l'élément filiforme 13 vers la tige 14 en métal élastique, d'un diamètre sensiblement égal ou légèrement supérieur au diamètre de l'élément filiforme 13 à un diamètre égal ou légèrement supérieur au diamètre de la tige 14 en métal élastique. Outre le fait qu'il constitue un moyen de sertissage de la tige 14 en métal élastique dans l'élément filiforme 13, un tel système de sertissage 22 permet de faciliter l'entrée dans les voies lacrymales de l'élément filiforme sans les blesser.

De préférence, le métal de la tige 14 en métal élastique est un acier inoxydable, en particulier présentant des propriétés super élastiques comme l'acier inoxydable vendu sous la dénomination commerciale « 304 » par la société UGINE. Alternativement, on peut utiliser comme métal élastique convenant à la tige du dispositif selon l'invention des alliages à base de cobalt, de chrome, de nickel et de molybdène, comme l'alliage à haute limite élastique vendu sous la dénomination commerciale « PHYNOX » par la société IMPHY, et présentant un module d'élasticité de 210 000 MPa, ou encore l'alliage vendu sous la dénomination commerciale « MP35N » par la société CARPENTER.

La figure 3b montre un exemple particulier de réalisation de la tige 14 en métal élastique présentant une portion rectiligne 20 et se prolongeant vers son extrémité distale par une portion courbée 21 comprenant l'extrémité distale courbée 15 de la tige 14. La partie courbée 21 est un arc de cercle qui présente par exemple un rayon de courbure R d'une valeur de 1 cm. Cette partie courbée 21 se termine par une excroissance 16 présentant un diamètre compris entre 0,04 cm et 0,06 cm. De préférence, le diamètre de l'excroissance 16 est supérieur au diamètre interne de la canule d'intubation 12. L'extrémité distale courbée 15 de la tige 14 en métal présente à son extrémité distale une excroissance 16, par exemple de diamètre 0,5 mm.

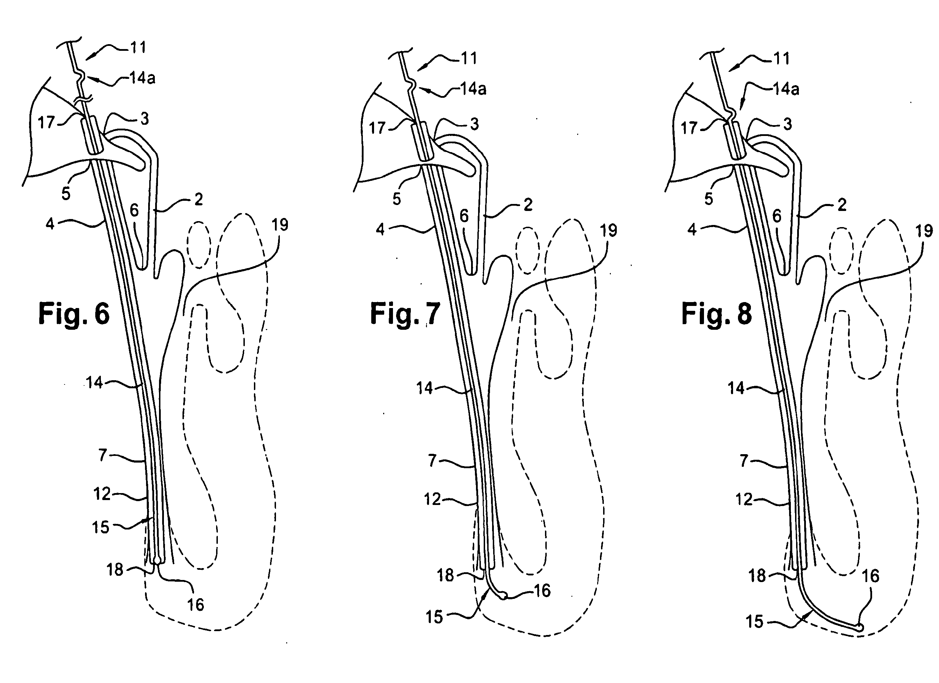

Ainsi, comme le montre la Figure 3a, l'extrémité distale courbée 15 de la tige en métal élastique 14 se déforme au moment où elle est totalement introduite dans la canule 12, sous la contrainte des parois de la canule qui tendent à aligner l'extrémité distale courbée 15 de la tige selon l'axe longitudinal de la canule 12, puis elle reprend sa forme initiale lorsqu'elle sort de la canule 12 comme montré sur les figures 7 et 8.

La tige 14 en métal présente par ailleurs dans sa partie proximale un décrochement 14a orienté dans la même direction que la courbure de l'extrémité distale courbée 15 de ladite tige 14. Ce décrochement 14a peut-être remplacé par tout autre dispositif ou marque permettant de connaitre l'orientation et la direction de la courbure de la tige 14 en métal élastique. A titre d'exemple, pour une tige 14 en métal élastique présentant une longueur totale de 21 cm, le décrochement est, par exemple, situé à environ 16 cm de l'extrémité distale de la tige 14 en métal élastique, et la canule d'intubation présente une longueur comprise entre 7 cm et 13 cm.

Avantageusement, la tige 14 en métal élastique a une longueur totale d'environ 18 cm. Ainsi, il est particulièrement aisé pour le chirurgien de récupérer l'extrémité distale courbée 15 de la tige 14, par exemple avec le doigt, lorsqu'elle sort de la fosse nasale puis de tirer dessus afin d'engager l'élément filiforme 13 dans un trajet lacrymal comme montré sur la figure 8. Alternativement, le chirurgien peut employer une pince pour récupérer l'extrémité distale courbée 15 de la tige 14 lorsqu'elle sort de la fosse nasale.

Afin d'assurer un coulissement correct, le diamètre de la tige 14 en métal élastique est inférieur au diamètre interne de la canule d'intubation. De préférence, le diamètre de la tige 14 en métal élastique est d'environ 0,25 mm.

De préférence, l'élément filiforme 13, par exemple le tube en silicone, a une longueur allant de 20 à 50 cm, de préférence allant de 20 à 30 cm. L'élément filiforme 13 peut être un tube plein ou un tube creux.

La canule d'intubation 12 peut présenter par exemple une longueur d'environ 7 cm. Elle présente un orifice distal 18a dont le diamètre interne est par exemple de 0,5 mm. Ainsi, le diamètre de l'excroissance 16 de l'extrémité distale courbée 15 de la tige 14 en métal élastique est supérieur ou égal à celui de l'orifice distal 18 de la canule 12.

Ainsi, la tige 14 en métal est solidaire de la canule 12 d'intubation. La tige 14 en métal élastique peut coulisser au sein de la canule d'intubation entre son excroissance 16 et son décrochement 14a. Dans l'exemple représenté, l'élément filiforme 13, la tige 14 en métal élastique et la canule d'intubation 12 sont indissociables, l'excroissance 16 de l'extrémité distale courbée 15, en forme par exemple d'un embout oliver, ayant un diamètre plus grand que le diamètre interne de la canule d'intubation, et d'autre part le décrochement 14a ne permettant pas la sortie complète de la tige 14 en métal élastique.

La Figure 3e représente un mode de réalisation de l'invention dans lequel la canule d'intubation 12 est courbée. La courbure de la canule d'intubation n'est pas nécessairement la même que celle de la tige 14 en métal élastique.

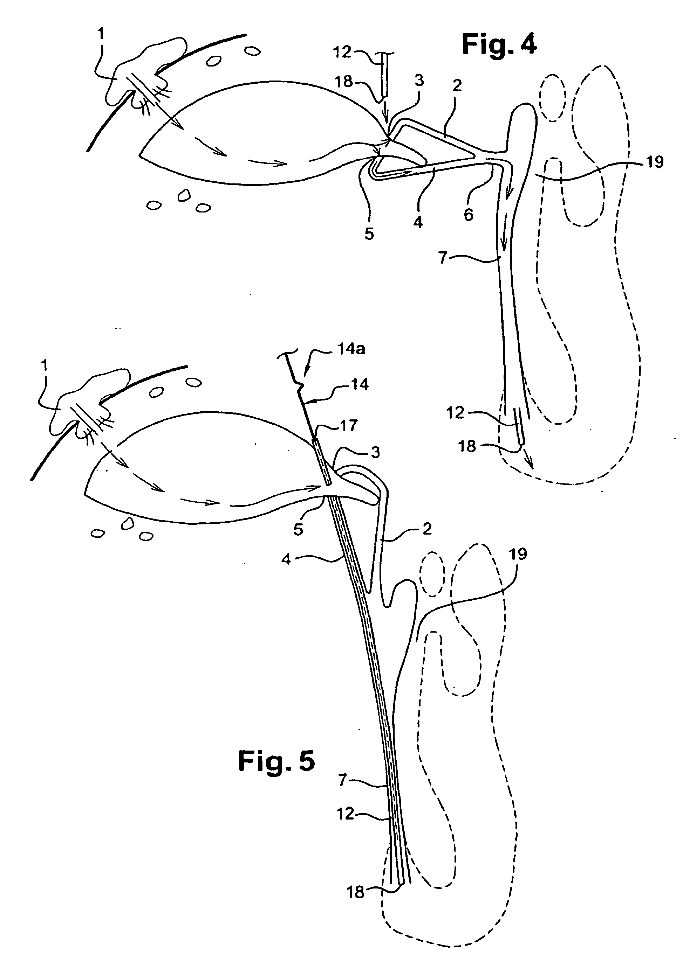

Sur la figure 4 est représenté le trajet emprunté par la canule 12 lors de l'intubation par le canalicule inférieur 6. L'extrémité distale 18 de la canule 12 est introduite dans le méat inférieur 5 puis elle est inclinée à 90° pour passer dans le canalicule inférieur 4. Lorsque l'extrémité distale 18 de la canule 12 bute contre l'os 19 du nez, la canule 12 est à nouveau inclinée à 90° pour la faire descendre dans le canal lacrymo-nasal 7 jusqu'au niveau de la fosse nasale. La canule 12 se trouve ainsi en position d'intubation, comme montré à la figure 5.

Lorsque la canule 12 est dans cette position d'intubation, on peut la retirer d'environ 1 cm, puis on fait glisser la tige 14 en métal élastique comme montré sur la figure 6. Comme on le voit sur la figure 5, l'extrémité distale courbée 15 de la tige 14 en métal élastique se déforme du fait de la contrainte exercée par les parois de la canule 12 et elle tend à s'aligner selon l'axe longitudinal de la canule 12.

En poussant la partie proximale de la tige 14 au niveau de l'extrémité proximale 17 de la canule 12, le chirurgien fait progresser lentement la tige 14 en métal élastique vers l'extrémité distale 18 de la canule 12 jusqu'à ce que l'extrémité distale courbée 15 de cette tige 14 sorte automatiquement en formant avec l'axe longitudinal de la canule 12 une courbure comme illustré sur la figure 7. En effet, dès que la contrainte des parois de la canule 12 ne s'exerce plus sur l'extrémité distale 15 de la tige 14 en métal élastique, cette extrémité distale 15 reprend sa forme initiale courbée et, grâce au coulissement exercé dans les parois inférieures des fosses nasales, elle débouche tout naturellement dans la fosse nasale.

Par ailleurs, grâce à l'orientation du décrochement 14a dans la même direction que la courbure de l'extrémité distale courbée 15 de la tige 14 en métal élastique, le chirurgien sait dans quelle direction l'extrémité distale courbée 15 va sortir dans la fosse nasale.

Le chirurgien continue alors à pousser sur la partie proximale de la tige 14 au niveau de l'extrémité proximale 17 de la canule 12 et l'extrémité distale courbée 15 de la tige 14 en métal élastique sort complètement de la fosse nasale comme illustré sur la figure 8. Le chirurgien peut alors récupérer facilement l'extrémité distale 15 de cette tige 14 en la saisissant avec le doigt. Alternativement audit doigt, il est envisageable d'utiliser une pince. Le chirurgien tire ensuite sur l'extrémité distale courbée 15 de cette tige 14 pour engager parfaitement l'élément filiforme 13 dans ce premier trajet lacrymal.

Dans le cas, non représenté, où l'on met en place un dispositif bicanaliculaire, le chirurgien retire la canule 12 de la première extrémité de l'élément filiforme 13 et les mêmes opérations que ci-dessus sont effectuées en introduisant la canule 12 de la deuxième extrémité de l'élément filiforme 13 par le méat supérieur 3 à travers le canalicule supérieur 2 puis le canal lacrymo-nasal. L'extrémité distale courbée 15 de la tige 14 en métal de la deuxième extrémité de l'élément filiforme 13 est récupérée facilement dans l'autre fosse nasale comme précédemment décrit.

Une fois les deux canules et tiges en métal sorties par les fosses nasales, les deux extrémités de l'élément filiforme sont sectionnées pour retirer les canules et tiges en métal élastique et les deux extrémités libres de l'élément filiforme sont nouées entre elles de façon classique afin de maintenir l'élément filiforme en place.

Le dispositif selon l'invention apporte une amélioration certaine aux dispositifs existants dans la mesure où il rend possible une pose de l'élément filiforme non traumatisante pour les chairs nasales des patients. L'intervention est également rendue plus simple pour le chirurgien. En particulier, grâce au dispositif selon l'invention, il est possible de poser une sonde monocanaliculaire ou bicanaliculaire en ambulatoire par exemple au cabinet du praticien.

## Revendications

1. Dispositif (11) pour le traitement du larmoiement de l'oeil, comprenant un élément filiforme (13) et une canule (12) d'intubation, au moins une extrémité de l'élément filiforme (13) étant munie d'une tige (14), ladite tige (14) étant agencée pour coulisser au sein de ladite canule d'intubation (12), **caractérisé en ce que** la tige est en métal élastique et comprend une extrémité distale courbée (15).

2. Dispositif (11) selon la revendication 1, **caractérisé en ce que** l'extrémité distale (15) de la tige (14) en métal élastique est courbée en arc de cercle.

3. Dispositif (11) selon la revendication 1 ou 2, **caractérisé en ce que** l'élément filiforme (13) est un tube de silicone.

4. Dispositif (11) selon l'une quelconque des revendications précédentes, **caractérisé en ce que** la tige (14) en métal élastique est constituée d'un acier inoxydable.

5. Dispositif (11) selon l'une quelconque des revendications précédentes, **caractérisé en ce que** la tige (14) en métal élastique est constituée d'un alliage à base de cobalt, de chrome, de nickel et de molybdène.

6. Dispositif (11) selon l'une quelconque des revendications précédentes, **caractérisé en ce que** l'élément filiforme (13) a une longueur allant de 20 à 50 cm, de préférence allant de 20 à 30 cm.

7. Dispositif (11) selon l'une quelconque des revendications précédentes, **caractérisé en ce que** la tige (14) en métal élastique a une longueur totale d'environ 18 cm.

8. Dispositif (11) selon l'une quelconque des revendications précédentes, **caractérisé en ce que** ladite canule (12) d'intubation est solidaire de ladite tige (14), en métal élastique.

9. Dispositif (11) selon la revendication précédente, **caractérisé en ce que** l'extrémité distale courbée (15) de la tige (14) en métal présente à son extrémité distale une excroissance (16) de diamètre supérieur ou égal au diamètre interne de l'orifice distal (18a) de la canule d'intubation (12).

10. Dispositif (11) selon l'une quelconque des revendications précédentes, **caractérisé en ce que** ladite tige (14) en métal élastique comprend dans sa partie proximale un décrochement (14a) orienté dans la même direction que la courbure de l'extrémité distale courbée (15) de ladite tige (14).

11. Dispositif (11) selon les revendications 8 ou 9, **caractérisé en ce que** la tige (14) en métal élastique est agencée pour coulisser au sein de ladite canule (12) entre ladite excroissance (16) et ledit décrochement (14a) seulement.

12. Dispositif (11) selon l'une quelconque des revendications précédentes, **caractérisé en ce que** chacune des deux extrémités (13a, 13b) de l'élément filiforme (13) est munie d'une tige (14) en métal élastique.

## Claims

1. A device (11) for treatment of watering of the eye, comprising a filamentary part (13) and an intubation cannula (12), at least one end of the filamentary part (13) being provided with a rod (14), said rod (14) being designed to slide inside said intubation cannula (12), **characterized in that** the rod is made of spring metal and has a curved distal end (15).

2. The device (11) as claimed in claim 1, **characterized in that** the distal end (15) of the spring metal rod (14) is curved into an arc of a circle.

3. The device (11) as claimed in claim 1 or 2, **characterized in that** the filamentary part (13) is a silicone tube.

4. The device (11) as claimed in any one of the preceding claims, **characterized in that** the spring metal rod (14) is made of a stainless steel.

5. The device (11) as claimed in any one of the preceding claims, **characterized in that** the spring metal rod (14) is made of an alloy based on cobalt, chromium, nickel and molybdenum.

6. The device (11) as claimed in any one of the preceding claims, **characterized in that** the filamentary part (13) is from 20 to 50 cm long, preferably from 20 to 30 cm long.

7. The device (11) as claimed in any one of the preceding claims, **characterized in that** the total length of the spring metal rod (14) is approximately 18 cm.

8. The device (11) as claimed in any one of the preceding claims, **characterized in that** said intubation cannula (12) is fixed to said spring metal rod (14).

9. The device (11) as claimed in the preceding claim, **characterized in that** the curved distal end (15) of the metal rod (14) has at its distal end an enlargement (16) whose diameter is greater than or equal to the internal diameter of the distal orifice (18a) of the intubation cannula (12).

10. The device (11) as claimed in any one of the preceding claims, **characterized in that** said spring metal rod (14) has in its proximal section a kink (14a) oriented in the same direction as the curvature of the curved distal end (15) of said rod (14).

11. The device (11) as claimed in claim 8 or 9, **characterized in that** the spring metal rod (14) is designed to slide inside said cannula (12) between said enlargement (16) and said kink (14a) only.

12. The device (11) as claimed in any one of the preceding claims, **characterized in that** each of the two ends (13a, 13b) of the filamentary part (13) is provided with a spring metal rod (14).

## Patentansprüche

1. Vorrichtung (11) zur Behandlung von tränendem Auge, die ein drahtförmiges Element (13) und eine Intubationskanüle (12) umfasst, wobei mindestens ein Ende des drahtförmigen Elements (13) mit einer Stange (14) ausgestattet ist, wobei die Stange (14) ausgebildet ist, um in der Intubationskanüle (12) zu gleiten, **dadurch gekennzeichnet, dass** die Stange aus elastischem Metall ist und ein gekrümmtes distales Ende (15) umfasst.

2. Vorrichtung (11) nach Anspruch 1, **dadurch gekennzeichnet, dass** das distale Ende (15) der elastischen Metallstange (14) kreisbogenförmig gekrümmt ist.

3. Vorrichtung (11) nach Anspruch 1 oder 2, **dadurch gekennzeichnet, dass** das drahtförmige Element (13) ein Silikonrohr ist.

4. Vorrichtung (11) nach einem der vorangehenden Ansprüche, **dadurch gekennzeichnet, dass** die elastische Metallstange (14) aus einem nichtrostenden Stahl besteht.

5. Vorrichtung (11) nach einem der vorangehenden Ansprüche, **dadurch gekennzeichnet, dass** die elastische Metallstange (14) aus einer Legierung auf der Basis von Kobalt, Chrom, Nickel und Molybdän besteht.

6. Vorrichtung (11) nach einem der vorangehenden Ansprüche, **dadurch gekennzeichnet, dass** das drahtförmige Element (13) eine Länge hat, die 20 bis 50 cm, vorzugsweise 20 bis 30 cm, beträgt.

7. Vorrichtung (11) nach einem der vorangehenden Ansprüche, **dadurch gekennzeichnet, dass** die elastische Metallstange (14) eine Gesamtlänge von zirka 18 cm hat.

8. Vorrichtung (11) nach einem der vorangehenden Ansprüche, **dadurch gekennzeichnet, dass** die Intubationskanüle (12) mit der elastischen Metallstange (14) verbunden ist.

9. Vorrichtung (11) nach vorangehendem Anspruch, **dadurch gekennzeichnet, dass** das gekrümmte distale Ende (15) der Metallstange (14) an seinem distalen Ende ein Verdickung (16) mit einem Durchmesser aufweist, der größer oder gleich dem Innendurchmesser der distalen Öffnung (18a) der Intubationskanüle (12) ist.

10. Vorrichtung (11) nach einem der vorangehenden Ansprüche, **dadurch gekennzeichnet, dass** die elastische Metallstange (14) in ihrem proximalen Abschnitt einen Knick (14a) aufweist, der in dieselbe Richtung wie die Krümmung des gekrümmten distalen Endes (15) der Stange (14) weist.

11. Vorrichtung (11) nach den Ansprüchen 8 oder 9, **dadurch gekennzeichnet, dass** die elastische Metallstange (14) ausgebildet ist, um in der Kanüle (12) nur zwischen der Verdickung (16) und dem Knick (14a) zu gleiten.

12. Vorrichtung (11) nach einem der vorangehenden Ansprüche, **dadurch gekennzeichnet, dass** jedes der zwei Enden (13a, 13b) des drahtförmigen Elements (13) mit einer elastischen Metallstange (14) ausgestattet ist.
